# EUROPEAN PATENT SPECIFICATION

(11) **EP 2 528 609 B1**
(45) Date of publication and mention of the grant of the patent: **23.08.2017**
(21) Application number: 11704839.7
(22) Date of filing: 28.01.2011
(51) Int. Cl.: A61K 35/64, A61L 15/40, A61K 9/00, A61P 17/02, A61L 31/16, A61K 9/06, A61K 9/70, A61K 47/32, A61L 24/00, A61K 35/644, A61K 36/00

(54) **PROPOLIS AND PROCESS FOR THE TREATMENT THEREOF AND END PRODUCTS FORMED THEREFROM**
PROPOLIS UND BEHANDLUNGSVERFAHREN DAFÜR SOWIE DARAUS HERGESTELLTE ENDPRODUKTE
PROPOLIS, SON PROCÉDÉ DE TRAITEMENT ET PRODUITS FINAUX OBTENUS

(30) Priority: 28.01.2010 GB 201001412
(43) Date of publication of application: 05.12.2012
(73) Proprietor: Natures Laboratory Limited, Whitby, North Yorkshire YO22 4NH (GB)
(72) Inventor: PARADKAR, Anant, Bradford West Yorkshire BD7 2AW (GB); DHUMAL, Ravindra, Bradford West Yorkshire BD8 9EX (GB); KELLY, Adrian, Bradford West Yorkshire BD14 6SN (GB); GILDA, Suhit, Satara 415 002 Maharashtra (IN)
(74) Representative: Wood, Graham
(86) International application number: PCT/GB2011/050147
(87) International publication number: WO 2011/092511

(56) References cited:
- WO-A2-00/24360
- KR-A- 20070 078 753
- US-A- 5 922 324
- US-A- 6 106 867
- US-A- 6 153 228
- AVANCO G B ET AL: "Preparation and characterisation of ethylcellulose microparticles containing propolis", REVISTA DE CIENCIAS FARMACEUTICAS BASICA E APLICADA 2008 UNIVERSIDADE ESTADUAL PAULISTA (UNESP) BRA, vol. 29, no. 2, 2008, pages 129-134, XP009148089, ISSN: 1808-4532
- BANKOVA V: "Recent trends and important developments in propolis research", EVIDENCE-BASED COMPLEMENTARY AND ALTERNATIVE MEDICINE 200503 GB LNKD- DOI:10.1093/ECAM/NEH059, vol. 2, no. 1, March 2005 (2005-03), pages 29-32, XP009148095, ISSN: 1741-427X
- GILDA S G ET AL: "Comparative evaluation of bioadhesive propolis gel (B-Gel) for mouth ulcers", JOURNAL OF PHARMACY AND PHARMACOLOGY, vol. 62, no. 10, Sp. Iss. SI, October 2010 (2010-10), pages 1255-1256, XP009148084, ISSN: 0022-3573

## Description

The present invention relates to a process for the treatment of propolis which allows the same to be more attractive for use in consumable goods and, in particular relates to a process which allows the deodorization of the propolis to be performed. In a further feature of the invention there is provided a process which can be used in addition to, or separately to, the deodorization process, which allows an improvement in the solubility of the propolis. The invention in a further aspect relates to propolis and, in particular, although not necessarily exclusively, to the provision of bioadhesive dosage forms of propolis in a manner so as to allow the delivery of the active constituents of the dosage to the desired site of action on a person or animal for a prolonged period of time. Examples of such sites could be sores, ulcers and/or thrush in oral cavity, external wounds and/or skin disorders. The dosage form in accordance with the invention can, in one embodiment, comprise of bioadhesive gels containing water soluble propolis and in another embodiment be in the form of bioadhesive patches of propolis produced from melt extruded, or casted, films. Preferably, although not necessarily exclusively, the propolis which is used to form the bioadhesive form of the same is that which has passed through the deodorization process of the invention

Propolis, sometimes also called "bee glue", is a strongly adhesive, resinous substance that honeybees collect from various plants, transformed and used by bees to seal holes in their honeycombs, to smooth out the beehive's internal walls and to protect the entrance against intruders (Burdock, 1995). Honeybees (*Apis mellifera* L.) collect the resin from cracks in plant barks and leaf buds. They masticate the resin and by doing so they add salivary enzymes to it. After this, they mix the partially digested material with beeswax and use it in their hive (Ghisalberti, 1979; Marcucci, 1995).

Propolis has been studied for various types of physiological effects such as antiinflammatory (Fitzpatrick, L et.al), antioxidant (Franciane D. Marquelea, et.al , Francesca Borrelli et.al), liver protective(Drogovoz SM, et.al), immunomodulatory (Jae Hyun Park), antimetastatic (Nada Oršolić et.al.), antimicrobial (Li-Chang Lu et.al), antibacterial (Abdul Al-Shaher et.al), antifungal, antiviral (Kujumgiev et.al) and for the treatment of chronic vaginal infection (M. Imhof and et.al). Propolis inhibited viral expression in a concentration-dependent manner (Genya Gekker). Caffeic acid phenethyl ester is one of the important compounds from propolis which has been investigated extensively for its bioactivity (Nicola Celli 2004). Several clinical trials demonstrating antimicrobial potential of propolis against highly resistant gram positive bacteria and yeast, (A. Russo, Fitoterpia, 2002, 73, S 21-9), oral infections, chronic vaginal infection, protective action against cartilage degeneration by IL 1 β (L. A. Doria, Italy life sci., 2003, 73, 1027-35). A number of properties have been attributed to propolis, and these include; bacteriostatic and bactericidal properties which are effective on a number of bacteria, including some staphylococci, streptococci and salmonella, *Bacillus subtills,* alvei and larvae; *Proteus vulgaris; Escherichia coli B.* The bactericidal effect is typically directly proportional to the concentration of the propolis. Fungicidal properties on some yeasts such as *Candida,* particularly *Candida albicans,* which can be responsible for creating mycosis or parasital ailments. Very powerful anaesthetic properties, three to four times more powerful than cocaine, which may be related to the volatile oils contained in propolis; and also the stimulating and regeneration of tissue.

A major problem with propolis is its poor water solubility which hampers its nutritional and therapeutic effectiveness. Few attempts have been reported to improve water solubility and availability of propolis such as:
US patent 5,922,324-1999 reports propolis extract with improved water-solubility using hydroalcoholic solution. It has been claimed for use in lotions, creams, injectables, shampoo, rinse, hair tonic, hair cream, tablet and toothpaste.

US patent 5,561,116-1996 reports Solid product containing propolis components, and preparation and uses thereof Solid, water-dispersible composition, with an aqueous solution of a water-soluble organic solvent mixing with anhydrous saccharides and cyclodextrins to dehydrate propolis Composition is suitable for use as a health food product, a sublingual agent.

CN patent 1685928-2005- Preparation method of nano propolis product. Nano-class propolis softgel, capsule or infusion using polyethanediol 6000 were prepared with stirring, adding lecithin, ultrasonic vibration, freeze drying, dissolving it in water, filtering by millipore membrane and free evaporating drying or freeze drying.

Polymeric nanoparticle encapsulated formulation of propolis (propolis nanofood) utilizing micellar aggregates of cross linked and random copolymers have been prepared to enable parenteral administration of propolis in an aqueous phase to explore anticancer potential in clinical area (Dong kim. 2008).

US patent 6,106,867-(2000) titled "Gelatinized propolis food products" claims about producing gel like food product of propolis, using a solution of gelatinizer as carragenan, xanthan gum, acacia, gelatin, dextran, PVA, PVP, CMC, HPMC etc.

US Patent 65915 describes a dentistry gel preparation "Propostom", the dentistry gel preparation comprising of phenolic hydrophobic active ingredient as propolis, ethanol, propylene glycol, carbopol, trometamol, and purified water, useful in treatment for dental decay and antibacterial effect.

US patent 5,942,244 "Local oral herbal slow release tablets". Tablets consist of polymeric matrix of ethyl cellulose, PEG 6000 as enhancer and lactose as filler.

CN1481822 and CN1528407-claims about Bee glue tablet for diabetes.

RU2237467 describes cream containing alcoholic solution of potentilla, capsicum and propolis, for massage, where cream is of pronounced anaesthetic, warming effect, increases circulation, decreases intumescences and degenerative alterations in tissues. Chinese patent CN1528175-2004 describes bee glue freeze-dried powder capsule and preparing method thereof.

Chinese patent CN1666666-2005 Propolis containing grape wine is described.

CN1493304-2004 mentions a method of extracting mixed propolis using supercritical fluid separation techniques.

CN1413598-2003 gives a manufacturing technology of nano bee glue Propolis and health-care food in the form of powder, capsule, tablet.

CN1711992-2005 explains about the production of bee-glue microcapsule with submicron softgels of propolis.

Propolis due to its tremendous therapeutic and prophylactic benefits is now considered to be an ingredient for fortified food and nutraceutical products. The use as a product of food composition mainly requires good palatability. Propolis due to its strong odour and solubility issues can not be incorporated easily in the food and nutraceutical products. Attempts to incorporate propolis in the products like honey and health drinks results in separation of phases, unpalatable, hazy product. The huge market potential is restricted due to these issues and therefore there is need to develop propolis in the water soluble and deodorized form.

There are number of methods reported for deodorization of some of the food ingredients and related substances. A number of patents are reported to remove odor of garlic by treatment using enzymes, fermentation (Japanese Patent Nos. 2,894/1960, 14,392/1963 and 27,308/1967); treatment using agents such as pyroligneous acid (Japanese Patent Application No. 19,936/1975); an aqueous solution of acetic acid (Japanese Patent Application No. 130,455/1978); phytic acid and silicic acid sol (Japanese Patent Application No. 29,265/1982); a menthol-containing solution (Japanese Patent Application No. 13,964/1989), etc. In addition removal of garlic odor constituents by treating garlic water extract with resins, active carbon or steam (see Japanese Patent Application Nos. 210,864/1984 and 100,259/1987), extraction with alcohol and the like, etc.

Deodorizing garlic by inactivating allinase, an enzyme involved in the formation of garlic odor, by heat inactivating the enzyme with a hot blast (see Japanese Patent Application Laid-open No. 77,560/1975), steam-cooking (see Japanese Patent Application Laid-open No. 198,065/1982), boiling (Japanese Patent Application No. 115,947/1976 and Japanese Patent Publication No. 12,658/1966), treatment using oil at high temperature (see Japanese Patent Application No. 28,658/1973), baking ( Japanese Patent Application No. 265,862/1989) and electromagnetic wave heating ( Japanese Patent Application Nos. 18,568/1973, 48,862/1974 and 164,762/1981). Deodorization of soyabean (US patent 4,076,851) where odor is emitted by soyabean fats is carried out by cooking of soyabean seed leaves for short period of time and removal of moisture by vacuum drying.

Other general methods of deodorization include use of adsorbents. As the deodorizer, an activated charcoal (mainly coconut shell activated charcoal) has mainly been used, but the activated charcoal has a disadvantage that it has a small deodorizing effect to a basic gas such as ammonia, alkyl amine, etc., while it has deodorizing effects to mercaptan, hydrogen sulfide, benzene, etc. Colloidal silica (silica sol) has an extremely larger specific surface area and can adsorb ammonia in the presence of water. Colloidal antimony pentoxide is capable of adsorbing and deodorizing various smells such as ammonia, amine, hydrogen sulfide, mercaptane, methyl sulfide, nicotine smell, etc.

There are no reports on either deodorization of propolis and or the deodorized water soluble propolis. This invention relates to development of novel deodorized and improved solubility form of propolis.

A first aim is therefore to provide a composition containing deodorized and/or improved palatability propolis in the water soluble form.

A further problem addressed by the current invention is to achieve an effective cure of intraoral conditions like ulcers, sores and thrush has been sought by the medical and dental communities without great success. There is essentially no known cause or cure of intraoral ulcers. These ulcers can be extremely painful to patients, and generally persist for seven to ten days. Historically, intraoral ulcers and lesions have been treated with chemical cautering techniques, often involving silver nitrate sticks. However there has been concern as to whether the pain caused by these treatments actually exceeds the pain caused by the ulcer or lesion they were intended to treat.

One commonly used treatment for intraoral ulcers is the use of acyclovir, an antiviral agent effective in treatment of certain forms of herpes. Acyclovir is available from Glaxo Wellcome under the tradename Zovirax. Zovirax consists essentially of acyclovir in a polyethylene glycol base and is available as an ointment or rinse. This product approaches the problem of ulcers based on the hypothesis that such ulcers or lesions are viral in nature. Another recent treatment is use of a product called Aphthasol, available from the Block Drug Company. Aphthasol consists of amlexanox, an antihistamine, in an adhesive paste. This treatment is based on the hypothesis that oral ulcers and lesions are caused by an autoimmune or allergic response of the body. However, none of the above products has proven to be effective in reliably reducing the pain associated with the ulcer while simultaneously speeding the healing process and preventing secondary infections.

Recently, a combination of antimicrobial agents, anti-inflammatories, and antihistamines are found to be effective in preventing both secondary infections and promoting healing while simultaneously providing immediate relief from pain and an example of this is disclosed United States Patent 6555125. However, the commonly used form of dosage to treat oral conditions are typically washed away by the saliva and therefore has limited impact and/or the patient has to apply the dosage frequently to the affected area.

Forms of treatment are also required for wounds, which can be internal or external bodily injuries or lesions caused by physical means, such as mechanical, chemical, viral, bacterial, or thermal means, which disrupt the normal continuity of structures. Such bodily injuries include, e.g., contusions, wounds in which the skin is unbroken, incisions, wounds in which the skin is broken by a cutting instrument, and lacerations, and/or wounds in which the skin is broken by a dull or blunt instrument.

A variety of products have been developed that enhance the body's ability to heal itself when wounded. These products typically function by medicating the wound, isolating the wound from infectious agents, and/or by binding the wound to prevent wound growth and to minimize the gap that the body's natural repair mechanisms must bridge to heal the wound.

Wound healing consists of a series of processes whereby injured tissue is repaired, specialized tissue is regenerated, and new tissue is reorganized. Wound healing consists of three major phases: a) an inflammation phase (0-3 days), b) a cellular proliferation phase (3-12 days), and (c) a remodeling phase (3 days-6 months).

The wounds can by nature, be contaminated and become infected and this interferes with the healing process and can often make the treatment more complex. Application of antimicrobial agents have significantly improved the healing process and reduced the healing time.

A further aim of the present invention is therefore to provide a dosage form of a propolis containing substance which allows a concentration of the active ingredients to be provided at the treatment site for a prolonged period of time.

In a first aspect of the invention there is provided a method for the removal of at least a portion of odoriferous and/or non palatable constituents from a propolis containing composition wherein said method includes the step of contacting propolis and water at a temperature in the range of 40-100 degrees Celsius for a predetermined period of time and characterised by removing at least one top floating layer of material from the water/propolis composition during said predetermined period of time.

In one embodiment the predetermined period of time is between 5 to 24 hours.

Typically the top floating layer of material is removed by a substantially continuous skimming to remove the floating layer and the supernatant which typically consists of the odoriferous and/or non palatable constituents followed by replacing the water.

Typically after the above steps the remaining propolis water mixture is dried. In one embodiment the drying is performed at a selected temperature, such as at 50°C.

In another embodiment of the present invention, the process of deodorizing and/or improving palatability may be performed at or below or above atmospheric pressures.

In another embodiment of the present invention, the process of deodorizing and/or improving palatability may be performed in the presence of deodorizing agents such as silicon dioxide, charcoal, zeolites.

In one embodiment the product is also treated to improve the solubility of the same to thereby aid the use of the propolis with other constituents.

In one embodiment the method includes the step of improving the water solubility of deodorized and/or improved palatability propolis by dispersing propolis in one or more solubility enhancer.

In one embodiment the dispersion is heated. Such dispersion, when added to an aqueous solvent readily disperses to yield a clear solution as observed with the naked eye. The clear aqueous solution is stable in a pH range of 3-9.

The solubility enhancer is preferably selected from Polysorbate derivatives, Polyoxyethylene castor oil derivatives, Polyhydroxy alcohols, Lecithin, Glyceryl monooleate, Glyceryl monosterate, Glyceryl palmitostearate, Glycofurol, Labrasol, well defined mixtures of mono-, di- and tri- glycerides and mono- and di-fatty acid esters of polyethylene glycol, Stearoyl Macrogolglycerides EP, Lauroyl Macrogolglycerides EP, α - Hydro - ω - hydroxyl - poly (oxy - 1, 2 - ethanediyl), sorbitol, Glycerol, Polyvinyl pyrolidone carrageenan, sugar, agar, starch, polyvinyl alcohol, polyacrylamide, polyethylene oxide, carboxymethyl cellulose, guar gum, xanthane gum, crosslinked polyacrylate, crosslinked polyacrylamide and mixtures thereof.

In an embodiment the improved solubility form comprises weight ratios of deodorized and/or improved palatability propolis to solubility enhancer in the range of about 1:0.001 or 0.001:1.

In another embodiment of the present invention, an improved solubility form of deodorized and/or improved palatability propolis has solubility more than about 10 folds to about 100 folds than its conventional form.

In a further embodiment the present invention also relates to use of the soluble form of deodorized and/or improved palatability propolis in consumable products such as pharmaceuticals, foodstuffs and/or cosmetics.

In a further aspect the present invention relates to a pharmaceutical composition comprising a soluble form of deodorized propolis and one or more pharmaceutically acceptable excipient formed using the method as herein described.

In accordance with the disclosure there is provided the use of a pharmaceutical composition comprising deodorized and/or improved palatability form of propolis for the treatment of various disease conditions in mammals.

In a yet further aspect of the invention there is provided a propolis product which has been processed to render the same substantially deodorized and/or improved palatability by contacting the propolis and water at 40-90 °C for 2 to 24 hours, with removal of the floating layer and the supernatant consisting of odoriferous and non palatable constituents followed by replacing the water and dried and wherein the floating layer is removed during the said period of time of 2 to 24 hours..

In one embodiment the propolis is obtained by dispersing the deodorized and/or palatability propolis in one or more solubility enhancer in a ratio in the range of 1:0.001 or 0.001:1 parts by weight and heating the dispersion from 40-90°C and which, upon addition to aqueous solvent readily disperses to yield a clear solution.

In one embodiment the process is performed in the presence of adsorbents such as silicon dioxide, charcoal, zeolites.

In one embodiment the solubility enhancer which is used is selected from a group comprising of Polysorbate derivatives, Polyoxyethylene castor oil derivatives, Polyhydroxy alcohols, Lecithin, Glyceryl monooleate, Glyceryl monosterate, Glyceryl palmitostearate, Glycofurol, Labrasol, well defined mixtures of mono-, di- and tri- glycerides and mono- and di-fatty acid esters of polyethylene glycol, Stearoyl Macrogolglycerides EP, Lauroyl Macrogolglycerides EP, α - Hydro - ω - hydroxyl - poly (oxy - 1, 2 - ethanediyl), sorbitol, Glycerol, Polyvinyl pyrolidone carrageenan, sugar, agar, starch, polyvinyl alcohol, polyacrylamide, polyethylene oxide, carboxymethyl cellulose, guar gum, xanthan gum, crosslinked polyacrylate, crosslinked polyacrylamide and mixtures thereof.

Preferably the majority of the odoriferous and/or non palatable constituents are removed to give the deodorized and/or improved palatability propolis.

In a further embodiment there is provided a bioadhesive composition including propolis and a bioadhesive agent in the form of gel or a patch in which odoriferous and/or non palatable constituents have been removed therefrom using the method as hereinbefore described.

In one embodiment, there is provided a bioadhesive composition that exhibits at least about 1100 mN strength of adhesion to a mucosal surface, when tested according to the test method described subsequently. Preferably the active constituents are released in a substantially continuous and sustained fashion over a prolonged period to a mucosal surface.

In one embodiment, the bioadhesive agents are selected from pharmaceutical grade hydrophilic or hydrophobic polymers, gums and/or resins.

In one embodiment, the polymer is selected from different grades of polyethylene oxide.

In one of the embodiments of the invention the bioadhesive gels are prepared by hydration of different grades of a carbomer, such as Carbopol and the dispersion of the propolis in the hydrated polymer followed by neutralization which, in turn, leads to the gelling of the composition.

In one of the embodiments of the invention the bioadhesive gels are prepared by hydration of different grades of pluronic and dispersion of propolis in the hydrated polymer to yield a bioadhesive gel.

In one of the embodiments of the invention the bioadhesive gels are prepared by hydration of a bioadhesive agent followed by the dispersion of the propolis to yield a bioadhesive gel.

In one of the embodiments of the invention bioadhesive patches are prepared from films obtained by the melt extrusion of a polymer and propolis blend.

In one of the embodiments of the invention the bioadhesive patches are prepared from films obtained by the melt casting of a polymer and propolis blend using a thermal press.

In one embodiment, the bioadhesive polymer is selected from any or any combination of, Poly (ethylene oxide), Carbopol (carbomer), poloxamer, polycarbophil, sodium carboxymethyl cellulose, hydroxypropyl cellulose, hydroxypropyl methyl cellulose, hydroxylethyl cellulose, carrageen, chitosan, , polylysine, polybrene, polyvinyl pyrrolidone, polyethylene glycol, hydroxylethyl methacrylate, poly (galacturonic acid), methyl cellulose and/or ethyl cellulose,

In one embodiment, the bioadhesive agent is selected from any, or any combination of, starch, dextran, gelatin, collagen, xanthan gum, xyloglucan gum, sclerohlucan, hyluronic acid, hyaluronan, hyaluronin, tragacanth, maltodextrin, pectin, alginic acid, alginates,

According to the disclosure there is provided a bioadhesive patch including propolis said patch provided to be attached on a person or animal in order to allow the delivery of active constituents to the site of action for a period of time.

According to the disclosure there is provided a bioadhesive gel including propolis said gel provided for application to a person or animal in order to allow delivery of active constituents to the site of action for a period of time.

According to the disclosure, the patch or gel, when adhered to the human mouth or external wounds, remains at the site of action, thereby releasing the active constituents in the form of propolis for a prolonged period of time and thereby allowing the healing effect of the same to be prolonged for a longer period of time than would conventionally be the case

According to the disclosure, the patch or gel is applied at the site of action on the human or animal so as to be used for treatment of oral ulcers, sores and/or thrush.

According to the disclosure, the patch or gel is applied at the site of action on the human or animal so as to be used for the treatment of external wounds and/or skin disorders.

In one embodiment the propolis used is a water soluble propolis, particularly when the propolis is to be provided as part of the gel composition.

In one embodiment the patch is formed by melt extrusion or melt casting. In one embodiment the patch is obtained from a film prepared by solvent evaporation, is obtained from the film prepared using plasticizer, and/or is prepared using 50 to 95% w/w polyethylene oxide as the bioadhesive film forming agent.

In one embodiment the gel is prepared using 50 to 95 % w/w of carbopol as the bioadhesive gelling agent.

In another embodiment the gel is prepared using 5 to 50 % w/w of pluronic as the bioadhesive gelling agent.

In one embodiment the bioadhesive agent is selected from any/or any combination of Poly (ethylene oxide), Carbopol (carbomer), poloxamer, polycarbophil, sodium carboxymethyl cellulose, hydroxypropyl cellulose, hydroxypropyl methyl cellulose, hydroxylethyl cellulose, carrageen, chitosan, , polylysine, polybrene, polyvinyl pyrrolidone, polyethylene glycol, hydroxylethyl methacrylate, poly (galacturonic acid), methyl cellulose, ethyl cellulose starch, dextran, gelatin, collagen, xanthan gum, xyloglucan gum, sclerohlucan, hyluronic acid, hyaluronan, hyaluronin, tragacanth, maltodextrin, pectin, alginic acid, and/or alginates.

Specific examples of the invention in practice are now provided for the purpose of illustration and without limiting the technical scope of the present invention;

### Example 1

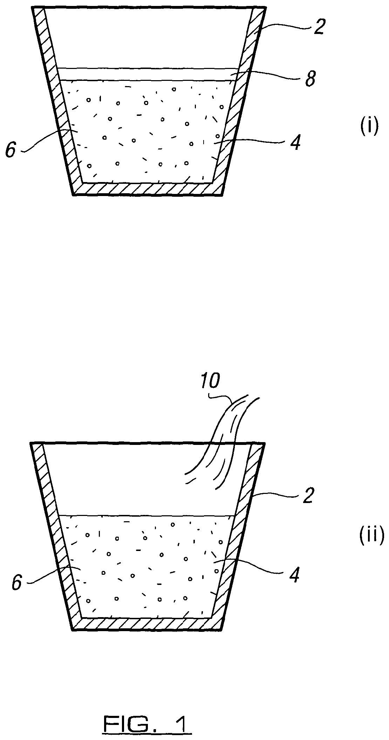

In the manufacture of deodorized Propolis in accordance with the invention and with reference to Figure 1, 500 g of propolis 4 was placed in a stainless steel vessel 2, and boiled along with water 6 at a ratio (1:10) at 70 °C for about 10 hours. After every 30 minutes a floating layer 8 and the supernatant water as shown in Figure 1 (i) formed and this was decanted and removed from the vessel 2 to bring the same to the condition shown in Figure 1(i). Fresh water 10 was added as shown in Figure 1(ii) with the water added typically being added in an amount to take up or replace the amount of the floating layer 8 that had been removed. This was repeated at 30 minutes intervals with successive floating layers 8 being removed and the steps (i) and (ii) being cycled through. Total yield after completion of process was 70%.

In order to evaluate the organoleptic properties of the product obtained by the invention a panel of 10 healthy volunteers (5 female and five male) from 18 to 40 years of age and body weight ranging from 60 to 80 kg participated in this study with informed consent. The volunteers were asked to smell and taste 100 mg of the treated propolis and untreated propolis powder. Taste evaluation began immediately after administration and continued for up to 2 minutes. A panel of 10 who tasted the treated propolis, judged it odorless and tasteless compared to untreated propolis which was judged to have irritating smell and very bitter taste.

### Example 2

In the manufacture of water soluble deodorized propolis, a number of surfactants as well as vehicles were screened and used for manufacture of water soluble propolis. The mixture of equal parts of deodorized and/or improved palatability propolis and polysorbate 80 were warmed at 60 °C to dissolve the propolis completely. The clear solution was transferred to vials, stoppered with rubber closure and aluminum cap was crimped. The vials were sterilized by steam sterilization.

### Example 3

In the manufacture of a deodorized and improved palatability water soluble propolis product, propolis was prepared as described in example 2 was mixed with 0.2 % of citric acid and heated until citric acid dissolved completely. Resultant mixture was allowed to cool with stirring at room temperature. Sucrose syrup at concentration of 17% was added to this solution and stirred homogeneously. Finally distilled water was added to make up the volume up to 100 ml.

### Formula

1. Deodorized water soluble propolis: 0.2% w/v
2. Sucrose syrup: 17 % v/v
3. Citric acid: 0.2% w/v
4. Distilled water: 82.6 %v/v

The invention therefore provides a propolis product which has been rendered substantially odourless and with improved palatability and optionally can be provided with improved solubility, thereby improving the utility of the propolis for use in end products and also increasing the scope of potential usage of the same as will now be described with reference to the following examples of end products.

### Example 4:

One possible end product is a bioadhesive patch and the following is an example of the manner in which the casting of bioadhesive propolis patches can be achieved.

Polyethylene glycol 400 was mixed with different grades of Polyethylene oxide in a mortar and pestle and blended with purified propolis in a turbula mixer, as shown in Table 1. An extruder with L:D ratio 40:1 and screw diameter 16 mm was used. The incorporated screw configuration consists of forward feeding elements and a small distributive mixing zone. The barrel temperature was set at 100 °C. Once the temperature was stabilized, the blend was fed to the extruder using a gravimetric twin-screw feeder (Brabender, Germany). The screw speed was set at 200 rpm. The resulting strand was collected at the extruder end and pelletized using a laboratory pelletizer (Eurolab, Varicut, UK).

A frame (10 x 10 Cm ²) was placed on the lower plate of a thermal press and a weighed amount of pellets were spread on a plate within the frame before placing the upper plate on top. The temperature of the thermal press was set at 105 °C and allowed to stabilize for 10 minutes. The plates were placed in the thermal press and pressure was gradually applied using the hydraulic press up to 2 tons over 2 minutes. After 1 minute the heaters were switched off and the press was cooled by circulating chilled water. Once the temperature reached 25 °C, the pressure was released. The plates were opened and the casted film removed carefully. The film was than cut into patches of desirable size, in accordance with the invention.

**Table 1: Composition of different propolis patches**

| **Composition** | **Batch numbers** | | | |
|---|---|---|---|---|
| | **1** | **2** | **3** | **4** |
| Propolis | 100 | 100 | 100 | 100 |
| Polyethylene oxide WSR N10 | 800 | - | - | - |
| Polyethylene oxide WSR N80 | - | 800 | - | - |
| Polyethylene oxide WSR N750 | - | - | 800 | - |
| Polyethylene oxide WSR Coagulant | - | - | - | 800 |
| Polyethylene glycol 400 | 50 | 50 | 50 | 50 |

The bioadhesion of all the films used to form patches in accordance with the invention was determined by the use of a tensiometric method. Freshly excised sheep intestinal tissue was stored in Tyrode's solution at 4°C and thawed to room temperature before use. The sheep intestinal tissue was cut into pieces (4 cm²), washed with cold Tyrode's solution, and blotted with tissue paper. The intestinal tissue was mounted on a stainless steel base of the apparatus with double-sided adhesive. The patch was placed on the lower side of the moving instrument probe of an advanced force gauge (Mecmesin, West Sussex, England), which was then lowered onto the tissue at a test speed of 0.5 mm/s so as to bring the tissue and sample into contact with each other. The experiments were performed at room temperature. The contact force was 200 mN, the contact time was 1 minute, and the probe was withdrawn at a rate of 0.1 mm/s. The peak detachment force was considered to be the bioadhesive force. All the batches showed a significant detachment force which was sufficient to indicate adequate bioadhesivity (Table 2). The bioadhesivity was found to increase with the molecular weight of the polyethylene oxide.

**Table 2. Detachment force for bioadhesive patches with different grades of polyethylene oxide**

| **Film** | **Force (mN)** |
|---|---|
| 1 | 1745 |
| 2 | 1930 |
| 3 | 2050 |
| 4 | 2390 |

In order to check the antimicrobial properties of the patch, a 10 mm² area from each film was cut apart and dissolved in 2 ml of sterile distilled water. The solution was stirred homogeneously and centrifuged at 10,000 rpm for 10 min and a supernatant was used for antimicrobial study against *E*. *coli, S. aureus and Candida albicans.* Microbial solutions were prepared at 10⁶ cells per ml concentrations and used as a control. In test samples the solutions were treated with the solutions of films from different batches and the UV absorbances were noted. The lower the absorbance value then the higher is the antimicrobial activity. All the batches showed significantly lowered absorbance values indicating significant antimicrobial activity as shown in Table 3.

**Table 3: Absorbances of the control and treated microbial solutions.**

| **Sample** | ***E. coli*** | ***S*. *aureus*** | ***Candida albicans*** |
|---|---|---|---|
| | Absorbance | Absorbance | Absorbance |
| Control | 0.363 | 0.695 | 0.246 |
| B. No. 1 | 0.024 | 0.095 | 0.046 |
| B. No. 2 | 0.019 | 0.089 | 0.063 |
| B. No. 3 | 0.018 | 0.065 | 0.063 |
| B. No. 4 | 0.022 | 0.078 | 0.055 |

### Example 5:

Another form of manufacture of bioadhesive propolis patches is now described with reference to the use of a single screw extruder.

Polyethylene glycol 400 was mixed with different grades of Polyethylene oxide in a mortar and pestle and blended with purified propolis in a turbula mixer. An extruder with an L:D ratio of 40:1 and screw diameter 16 mm was used. The incorporated screw configuration consists of forward feeding elements and a small distributive mixing zone. The barrel temperature was set at 100 °C and the screw speed at 200 rpm. Once the temperature was stabilized, the blend was fed to the extruder using a gravimetric twin-screw feeder (Brabender, Germany).. The resulting strand was collected at the extruder end and pelletized using a laboratory pelletizer (Eurolab, Varicut, UK).

The pellets were fed to the single screw extruder fitted with a slit die set at 105 °C and screw speed of 200 rpm. The material was extruded to produce a film, 2.5 cm wide and 0.5 cm thick. The film was then cut into patches of desirable size.

### Example 6:

A yet further example of the manufacture of bioadhesive propolis patches using a twin screw extruder and chill roller is now described.

Polyethylene glycol 400 was mixed with different grades of Polyethylene oxide in a mortar and pestle and blended with purified propolis in a turbula mixer. An extruder with an L:D ratio 40:1 and a screw diameter of 16 mm was used with a split die. The incorporated screw configuration consisted of forward feeding elements and a small distributive mixing zone. The barrel temperature was set at 100 °C and the speed at 200 rpm. Once the temperature was stabilized, the blend was fed to the extruder using a gravimetric twin-screw feeder (Brabender, Germany). The resulting film was guided through the chill roller to cause the calendaring into thin uniform film, 2.5 cm wide and 0.5 cm thick. The film was than cut into patches of desirable size.

### Example 7

Another example of an end product which can be formed is a gel and a manufacturing method in accordance with the invention is now described for a bioadhesive propolis gel.

2 gm carbopol 934 NF was dispersed uniformly in 90 ml distilled water with constant stirring at 50 rpm. The stirring was continued until all carbopol was hydrated properly. A quantity of 1.5 gm of water soluble propolis was dissolved in 10 ml water and added to the hydrated carbopol solution with constant stirring. The pH was adjusted to 7 with triethanolamine solution to create a transparent propolis gel.

In order to test the antimicrobial activity of the bioadhesive propolis gel, the gel was dissolved in sterile distilled water, stirred homogeneously, centrifuged at 10,000 rpm for 10 min and a supernatant was used for a antimicrobial study against *E*. *coli, S. aureus and Candida albicans.* Microbial solutions were prepared at 10⁶ cells per ml concentrations and used as control. Samples with different concentrations of propolis gel, and commercial gel having choline salicylate were prepared and the UV absorbance was noted. The lower the value of the absorbance the higher is the antimicrobial activity. The results show that antimicrobial activity was concentration dependent and improved with the concentration for both the propolis gel as well as commercial gel (Table 4).

**Table 4: Absorbance's of the control and treated microbial solutions.**

| ***S. aureus*** | **Concentration(ug/ml)** | **Propolis gel** | **Bonjela gel** | **Control (10⁶ cells/ml)** |
|---|---|---|---|---|
| | - | - | - | 0.695 |
| | 100 | 0.305 | 0.270 | - |
| | 200 | 0.240 | 0.198 | - |
| | 300 | 0.148 | 0.132 | - |
| | 400 | 0.110 | 0.089 | - |
| | 500 | 0.099 | 0.086 | - |

| ***E. coli*** | **Concentration(ug/ml)** | **Propolis gel** | **Bonjela gel** | **Control (10⁶ cells/ml)** |
|---|---|---|---|---|
| | - | - | | 0.363 |
| | 100 | 0.082 | 0.073 | - |
| | 200 | 0.049 | 0.036 | - |
| | 300 | 0.034 | 0.023 | - |
| | 400 | 0.018 | 0.011 | - |
| | 500 | 0.019 | 0.00 | - |

| ***Candida albicans*** | **Concentration(ug**/**ml**) | **Propolis gel** | **Bonjela gel** | **Control (10⁶ cells/ml)** |
|---|---|---|---|---|
| | - | - | - | 0.246 |
| | 100 | 0.131 | 0.103 | - |
| | 200 | 0.095 | 0.077 | - |
| | 300 | 0.091 | 0.080 | - |
| | 400 | 0.092 | 0.065 | - |
| | 500 | 0.090 | 0.063 | - |

The Bioadhesion of all the gel was determined by the tensiometric method. Freshly excised sheep intestinal tissue was stored in Tyrode's solution at 4-C and thawed to room temperature before use. The sheep intestinal tissue was cut into pieces (4 cm²), washed with cold Tyrode's solution, and blotted with tissue paper. The intestinal tissue was mounted on a stainless steel base of the apparatus with double-sided adhesive. The gel was spread on the lower side of the moving instrument probe of the advanced force gauge (Mecmesin, West Sussex, England), which was then lowered onto the tissue at a test speed of 0.5 mm/s so as to bring the tissue and sample into contact with each other. The experiments were performed at room temperature. The contact force was 0.2 N, the contact time was 1 minute, and the probe was withdrawn at a rate of 0.1 mm/s. The peak detachment force was considered to be the bioadhesive force. The gel showed significant detachment force (1360 mN) which indicated an improved and adequate bioadhesivity compared to the value of 930 mN for the commercial gel.

The anesthetic activity of the bioadhesive propolis gel prepared in accordance with the invention was tested on groups of male Wistar rats (130 to 175 g). To induce inflammation, 0.1 ml of a 1% suspension of carrageen in distilled water was injected subcutaneously into the plantar surface of the hind paw of the rat. 1 gm sample of each gel was applied to foot. Three hours later, pressure was applied through a tip to the plantar surface of the rat's foot at a constant rate by a special apparatus to the point at which the animal struggles, squeals or attempts to bite. The force was applied to the animal's paw, which was placed on a small plinth under a cone-shaped pusher with a rounded tip. The operator depressed a pedal-switch to start the mechanism which exerts the force. When the rat struggles, the operator releases the pedal and reads off the scale the force at which the animal struggled. A control animal showed a displacement of 7.5 cm. The anesthetic activity of the propolis gel was compared with two commercial formulations. The Propolis gel showed better anesthetic activity than the commercial formulations (Table 5).

**Table 5. Anesthetic activity of commercial gels and propolis bioadhesive gel**

| **Group** | **Displacement** | **AVG ± SD** | **% anesthetic** |
|---|---|---|---|
| **Commercial gel containing choline salicylate** | 10 cm | 10.875 ± 2.096 | **45%** |
| | 9.5 cm | | |
| | 10 cm | | |
| | 14 cm | | |
| **Commercial gel containing Cetylpyridinium Chloride and Lidocaine** | 11 cm | 11.25 ± 1.258 | **50%** |
| | 13 cm | | |
| | 10 cm | | |
| | 11 cm | | |
| **Bioadhesive propolis gel** | 12 cm | 12.125 ± 1.652 | **61**.**66%** |
| | 10 cm | | |
| | 14 cm | | |
| | 12.5 cm | | |

The present invention therefore provides a means whereby the benefits and advantageous characteristics of propolis can be provided as part of a carrier in the form of a gel or patch which allows the propolis to be located at an active site at which the propolis is exposed to the particular condition of the person or animal to which the gel or patch is applied. Although not specifically required in order to allow the advantages of the propolis to be achieved it is preferable that the propolis has been deodourised and/or made more palatable and/or more soluble using the processing method described in this document prior to being adapted into the end product.

## Claims

1. A method for the removal of at least a portion of odoriferous and/or non palatable constituents from a propolis containing composition wherein said method includes the step of contacting propolis (4) and water (10) at a temperature in the range of 40-100 °C for a predetermined period of time and **characterised by** removing at least one top floating layer (8)of material from the water/propolis composition during said predetermined period of time.

2. A method according to claim 1 wherein the predetermined period of time is between 5 to 24 hours.

3. A method according to claim 1 wherein the removal is performed at regular intervals or substantially continuously by skimming to remove any floating layer of material and the supernatant which consists of the odoriferous and/or non palatable constituents.

4. A method according to claim 1 wherein the removal of material is followed by adding water to replace the material which has been removed.

5. A method according to claim1 wherein the method is performed in the presence of deodorizing agents.

6. A method according to any of the preceding claims wherein the method includes the step of dispersing propolis in one or more solubility enhancer.

7. A method according to claim 6 wherein the dispersion is heated and added to an aqueous solvent to yield a substantially clear solution composition.

8. A pharmaceutical product comprising a propolis composition formed using the method of claims 1 -8 and one or more pharmaceutically acceptable excipient.

9. A propolis product wherein the propolis is processed by contacting the propolis (4) with water (10) at a temperature of between 40-90 °C for 2 to 24 hours, and removing a floating layer (8) including a supernatant consisting of odoriferous and/or non palatable constituents, replacing the water and drying to form the said propolis product and wherein the floating layer (8) is removed during the said period of time of 2 to 24 hours.

10. A bioadhesive composition including a propolis composition and a bioadhesive agent provided in the form of gel or a patch wherein the propolis composition has had odoriferous and/or non palatable constituents removed therefrom using the method of any of claims 1-8.

11. A composition according to claim 10 wherein the bioadhesive composition exhibits at least 1100 mN strength of adhesion to a mucosal surface.

12. A composition according to claim 10 wherein the active constituents therein are released in a substantially continuous and sustained fashion over a period of time to a mucosal surface with which the composition contacts.

13. A composition according to claim 10 wherein the bioadhesive agents are selected from pharmaceutical grade hydrophilic or hydrophobic polymers, gums and/or resins.

14. A method of forming a gel of the composition defined in claims 10-11 wherein the bioadhesive gel is prepared by hydration of one or more grades of a carbomer, and the dispersion of the propolis in the hydrated polymer followed by neutralization of the gel of the composition.

15. A method of forming a gel of the composition as defined in any of claims 10-11 wherein a bioadhesive agent is hydrated followed by the dispersion of the propolis in the same to yield a bioadhesive gel.

## Patentansprüche

1. Verfahren zum Entfernen wenigstens eines Teils von riechenden und/oder nicht schmackhaften Bestandteilen aus einer Propolis enthaltenden Zusammensetzung, wobei das genannte Verfahren den Schritt des Inkontaktbringens von Propolis (4) und Wasser (10) bei einer Temperatur im Bereich von 40-100°C für eine vorbestimmte Zeitperiode beinhaltet, und **gekennzeichnet durch** Entfernen von wenigstens einer oberen schwimmenden Materialschicht (8) von der Wasser/Propolis-Zusammensetzung während der genannten vorbestimmten Zeitperiode.

2. Verfahren nach Anspruch 1, wobei die vorbestimmte Zeitperiode zwischen 5 und 24 Stunden beträgt.

3. Verfahren nach Anspruch 1, wobei das Entfernen in regelmäßigen Abständen oder im Wesentlichen kontinuierlich durch Abschöpfen erfolgt, um eine schwimmende Materialschicht und den aus den riechenden und/oder nicht schmackhaften Bestandteilen bestehenden Überstand zu entfernen.

4. Verfahren nach Anspruch 1, wobei auf das Entfernen von Material das Zugeben von Wasser folgt, um das Material zu ersetzen, das entfernt wurde.

5. Verfahren nach Anspruch 1, wobei das Verfahren in Anwesenheit von desodorierenden Mitteln durchgeführt wird.

6. Verfahren nach einem der vorherigen Ansprüche, wobei das Verfahren den Schritt des Dispergierens von Propolis in einem oder mehreren Löslichkeitsverbesserern beinhaltet.

7. Verfahren nach Anspruch 6, wobei die Dispersion erhitzt und einem wässrigen Lösungsmittel zugegeben wird, um eine im Wesentlichen klare Lösungszusammensetzung zu erhalten.

8. Pharmazeutisches Produkt, das eine Propoliszusammensetzung umfasst, hergestellt mit dem Verfahren nach Anspruch 1-8, und ein oder mehrere pharmazeutisch akzeptable Hilfsstoffe.

9. Propolisprodukt, wobei die Propolis verarbeitet wird durch Inkontaktbringen der Propolis (4) mit Wasser (10) bei einer Temperatur zwischen 40 und 90°C für 2 bis 24 Stunden und Entfernen einer schwimmenden Schicht (8) einschließlich eines Überstands bestehend aus riechenden und/oder nicht schmackhaften Bestandteilen, Ersetzen des Wassers und Trocknen zum Bilden des genannten Propolisprodukts, und wobei die schwimmende Schicht (8) während der genannten Zeitperiode von 2 bis 24 Stunden entfernt wird.

10. Bioadhäsive Zusammensetzung, die eine Propoliszusammensetzung und ein bioadhäsives Mittel enthält, bereitgestellt in Form von Gel oder eines Patch, wobei riechende und/oder nicht schmackhafte Bestandteile mit dem Verfahren nach einem der Ansprüche 1-8 aus der Propoliszusammensetzung entfernt wurden.

11. Zusammensetzung nach Anspruch 10, wobei die bioadhäsive Zusammensetzung wenigstens 1100 mN Haftstärke auf einer Schleimhautoberfläche aufweist.

12. Zusammensetzung nach Anspruch 10, wobei die aktiven Bestandteile darin auf eine im Wesentlichen kontinuierliche und anhaltende Weise über eine Zeitperiode auf eine Schleimhautoberfläche freigesetzt werden, mit der die Zusammensetzung in Kontakt kommt.

13. Zusammensetzung nach Anspruch 10, wobei die bioadhäsiven Mittel ausgewählt sind aus hydrophilen oder hydrophoben Polymeren, Gummis und/oder Harzen von pharmazeutischer Güte.

14. Verfahren zum Bilden eines Gels der Zusammensetzung gemäß Definition in den Ansprüchen 10-11, wobei das bioadhäsive Gel durch Hydratisieren von einer oder mehreren Güten eines Carbomers und Dispergieren der Propolis in dem hydratisierten Polymer gefolgt vom Neutralisieren des Gels der Zusammensetzung hergestellt wird.

15. Verfahren zum Bilden eines Gels der Zusammensetzung gemäß Definition in einem der Ansprüche 10-11, wobei ein bioadhäsives Mittel hydratisiert wird, gefolgt vom Dispergieren der Propolis darin, um ein bioadhäsives Gel zu erhalten.

## Revendications

1. Procédé d'extraction d'au moins une partie de constituants odoriférants et/ou non sapides d'une composition contenant de la propolis, ledit procédé incluant l'étape de mise en contact de la propolis (4) et de l'eau (10) à une température comprise dans l'intervalle de 40 à 100 °C pendant une période de temps prédéterminée et **caractérisé par** l'extraction d'au moins une couche flottante supérieure (8) de matériau de la composition d'eau/propolis au cours de ladite période de temps prédéterminée.

2. Procédé selon la revendication 1 dans lequel la période temps prédéterminée est de 5 à 24 heures.

3. Procédé selon la revendication 1 dans lequel l'extraction est réalisée à intervalles réguliers ou substantiellement de façon continue en écumant afin d'éliminer toute couche flottante de matériau et le surnageant qui consiste en des constituants odoriférants et/ou non sapides.

4. Procédé selon la revendication 1 dans lequel l'extraction des matériaux est suivie par l'addition d'eau pour remplacer le matériau qui a été extrait.

5. Procédé selon la revendication 1 dans lequel ledit procédé est réalisé en présence d'agents désodorisants.

6. Procédé selon l'une quelconque des revendications précédentes dans lequel ledit procédé inclut l'étape de dispersion de la propolis dans un ou plusieurs promoteurs de solubilité.

7. Procédé selon la revendication 6 dans lequel la dispersion est chauffée et ajoutée à un solvant aqueux de manière à obtenir une composition de solution substantiellement limpide.

8. Produit pharmaceutique comprenant une composition de propolis formée en utilisant le procédé des revendications 1 à 8 et un ou plusieurs excipients pharmaceutiquement acceptables.

9. Produit propolis dans lequel la propolis est traitée par mise en contact de la propolis (4) avec de l'eau (10) à une température comprise entre 40 et 90 °C pendant 2 à 24 heures, et par extraction d'une couche flottante (8) contenant un surnageant constitué de constituants odoriférants et/ou non sapides, par remplacement de l'eau et séchage de manière à former ledit produit propolis et dans lequel la couche flottante (8) est extraite au cours de ladite période de temps de 2 à 24 heures.

10. Composition bioadhésive contenant une composition de propolis et un agent bioadhésif fourni sous forme de gel ou d'un timbre, de laquelle composition de propolis des constituants odoriférants et/ou non sapides ont été extraits en utilisant le procédé selon l'une quelconque des revendications 1 à 8.

11. Composition selon la revendication 10 dans laquelle le composition adhésive fait preuve d'une force d'adhérence d'au moins 1100 mN à une surface de muqueuses.

12. Composition selon la revendication 10 dans laquelle les constituants actifs qui y sont contenus sont libérés d'une façon substantiellement continue et constante au cours d'une période de temps sur une surface de muqueuses avec laquelle la composition est mise en contact.

13. Composition selon la revendication 10 dans laquelle les agents bioadhésifs sont sélectionnés à partir de polymères hydrophiles ou hydrophobes, de gommes et/ou de résines de qualité pharmaceutique.

14. Procédé de formation d'un gel de la composition définie dans les revendications 10 à 11 dans lequel le gel bioadhésif est préparé par hydratation d'un ou de plusieurs grades d'un carbomère, et la dispersion de la propolis dans le polymère hydraté est suivie par la neutralisation du gel de la composition.

15. Procédé de formation d'un gel de la composition telle que définie dans l'une quelconque des revendications 10 à 11 dans lequel l'agent bioadhésif est hydraté, puis la propolis est dispersée dans celui-ci pour obtenir un gel bioadhésif.
